# EUROPEAN PATENT APPLICATION

(11) **EP 4 140 474 A1**
(43) Date of publication of application: **01.03.2023**
(21) Application number: 21000240.8
(22) Date of filing: 23.08.2021
(51) Int. Cl.: A61K 9/00, A61K 31/05, A61K 31/12, A61K 31/216, A61K 31/355, A61K 31/375, A61K 33/06, A61K 33/30, A61K 36/00, A61P 19/02, A61P 29/00

(54) **NUTRACEUTICAL FOR THE REDUCTION OF OSTEOARTHRITIC PAIN AND INFLAMMATION**

(71) Applicant: Theracell Advanced Biotechnology S.A., 145 64 N. Kifisia Attikis (GR); Cloudpharm Private Company, 151 25 Marousi Attikis (GR)
(72) Inventor: Sakellaridis, Fotlos, GR-145 64 N. Kifisia Attikis (GR)
(74) Representative: Roukounas, Dimitrios

(57) **Abstract**

The present invention relates to a pharmaceutical composition or dietary supplement composition or nutraceutical composition for the reduction of osteoarthritic pain and inflammation in general, and in particular the present invention relates to a pharmaceutical composition or dietary supplement composition or nutraceutical comprising a therapeutically effective amount of resveratrol or a pharmaceutically acceptable derivative thereof in combination with a therapeutically effective amount of rosmarinic acid and at least another one compound, such as curcumin to be used for the reduction of osteoarthritic pain and inflammation, and a process for the preparation of said composition.

## Description

### TECHNICAL FIELD OF THE INVENTION

The present invention relates to a pharmaceutical composition or dietary supplement composition or nutraceutical composition for the reduction of osteoarthritic pain and inflammation in general, and in particular the present invention relates to a pharmaceutical composition or dietary supplement composition or nutraceutical comprising a therapeutically effective amount of resveratrol or a pharmaceutically acceptable derivative thereof in combination with a therapeutically effective amount of rosmarinic acid and at least another one compound, such as curcumin, to be used for the reduction of osteoarthritic pain and inflammation, and a process for the preparation of said composition.

### BACKGROUND OF THE INVENTION

Osteoarthritis (OA) is a degenerative arthropathy and chronic disorder. It is a multifactorial disease and the most common form of arthritis. It belongs to one of the inflammatory diseases and is manifested with significant loss of cartilage and narrowing of the mesarter. Symptomatic OA of the joints has adverse effects on society and the economy, as it is the third most common cause of long-term functional disability, after the group of other rheumatic diseases and the group of cardiovascular diseases. In our country, the total prevalence of symptomatic OA is 13.1% of the adult population.

Symptoms such as pain, stiffness, and gelling in OA contribute to patients' functional limitations, with a well-documented correlation between pain level and degree of functional limitation. In Greece, the knee joint is the most common localization of symptomatic OA, with approximately 20% of patients with symptomatic osteoarthritis presenting two or more localizations.
In terms of treatment costs, OA was ranked as the fifth most expensive treatment in the United States in 2008, costing about $ 40 billion in all national hospitals. The estimated number of adults in the USA with a documented diagnosis of arthritis, mainly with a diagnosis of OA, is projected to increase to approximately 67 x 10⁶ by 2030.
The symptoms of osteoarthritis are the breakdown of articular cartilage accompanied by inflammation, tingling, stiffness, deformities and joint pain.

The mechanisms involved in the inflammatory response to osteoarthritis are summarized in the following.
1. The progression of osteoarthritis alters the production and function of certain cytokines. In particular, (a) the inflammatory interleukins IL-1β, IL-6, IL-15, IL-17, and IL-18, (b) the anti-inflammatory interleukins IL-4, IL-10, and IL-13, and (c) tumor necrosis factor α (TNF-α).
2. The enzymes COX-1 and especially COX-2 are responsible for the biosynthesis of prostaglandin E2 (PGE2) which reduces pain levels. COX-2 production is overexpressed by proinflammatory cytokines such as IL-1 as well as factor TNF-α.
3. Uterine metalloproteinase-13 (MMP-13) is specifically expressed in the joint of patients with osteoarthritis and is the main joint collagenase with the highest activity against type II collagen.
4. Abnormal metabolism of chondrocytes leads to the production of a large concentration of free radicals that exceed the regulatory antioxidant capacity. Free radicals are important signaling molecular species that enhance the inflammatory response. They can also induce structural and functional changes in the extracellular matrix leading to joint stiffness and fragility in patients with OA.
5. The enzyme 5LOX lipoxygenase and the P2X7 receptor are generally involved in the development of inflammatory arthritis and rheumatoid arthritis.

The first treatment approach is based on non-medication (no pharmaceutical treatment) that includes weight loss, rest and physiotherapy.

The pharmaceutical treatment in OA is symptomatic and involves the administration of analgesic and non-steroidal anti-inflammatory drugs (NSAIDs) to treat the onset of inflammation. In this regard, there are many treatments that can mainly relieve pain and inflammation. One treatment involves the use of non-steroidal anti-inflammatory drugs (NSAIDs) such as aspirin, ibuprofen, acetaminophen, naproxen and diclofenac. Although NSAIDs relieve some stiffness, inflammation, and pain, NSAIDs can lead to side effects such as gastric bleeding, liver damage, and kidney damage.
In addition, long-term use of NSAIDs can lead to reduced effectiveness. Further, the treatment is restricted to alleviation of the symptoms and does nothing to improve the underlying condition.

Supplements comprising bioactive ingredients with known anti-inflammatory properties have been shown to reduce the risk of developing OA of the knee and include, for example, vitamin C, vitamin E, β-carotene as well as extracts. Similarly, some flavonoids have been shown to have antioxidant and anti-inflammatory activity in OA, inhibiting the enzymes lipoxygenase and cyclooxygenase.

Therefore, despite the progress in the understanding of the pathophysiology of osteoarthritic pain and inflammation, and the development of new treatment protocols, there is a need for a drug treatment that is effective and well tolerated without undesirable side effects.

### SUMMARY OF THE INVENTION

It is, therefore, an object of the present invention to provide a pharmaceutical composition or dietary supplement composition or nutraceutical for the reduction of osteoarthritic pain and inflammation, which overcomes the deficiencies of the prior art.

It is another object of the present invention to provide a pharmaceutical composition or dietary supplement composition or nutraceutical for the reduction of osteoarthritic pain and inflammation, which is safe and effective with no side effects and good pharmacotechnical properties.

Moreover, it is another object of the present invention to provide a suitable process for the preparation of a pharmaceutical composition or dietary supplement composition or nutraceutical for oral administration, which is effective and reproducible.

In accordance with the above objects of the present invention, a pharmaceutical composition or dietary supplement composition or nutraceutical is provided comprising a therapeutically effective amount of resveratrol or a pharmaceutically acceptable derivative thereof, in combination with a therapeutically effective amount of rosemarinic acid and at least one additional compound, such as curcumin, for the reduction of osteoarthritic pain and inflammation.

According to another embodiment of the present invention, a process for the preparation of a pharmaceutical composition or dietary supplement composition or nutraceutical comprising a therapeutically effective amount of resveratrol or a pharmaceutically acceptable derivative thereof, in combination with a therapeutically effective amount of rosmarinic acid and at least one additional compound, such as curcumin, for the reduction of osteoarthritic pain and inflammation is provided, wherein said process comprises following steps:
a) Weighing the individual active substances and their extracts, namely Polygonum Cuspidatum extract, rosemary extract, turmeric, ascorbic acid, and excipients, such as magnesium stearate and microcrystalline cellulose.
b) Mixing all said active substances together with the excipients, such as magnesium stearate and microcrystalline cellulose for appropiate time so as to form a homogenous mixture.
c) Adding all said active substances in a blender and mixing for appropiate time until completely uniform.
d) Filling hard capsules or sachets
e) Packaging and quality control.

Further preferred embodiments of the present invention are defined in dependent claims 2 to 7 and 9.
Other objects and advantages of the present invention will become apparent to those skilled in the art in view of the following detailed description.

### DETAILED DESCRIPTION OF THE INVENTION

It has been surprisingly found that the object of the present invention is achieved by employing resveratrol or a pharmaceutically acceptable derivative thereof due to its anti-inflammatory and analgesic properties, in combination with rosmarinic acid and at least one additional compound such as curcumin, which are potent antioxidants for the reduction of osteoarthritic pain and inflammation.

The active ingredients used in the present invention have anti-inflammatory properties against biological mechanisms of osteoarthritis. Therefore, they support the reduction of inflammation in the joint and consequently the pain.

Resveratrol is a plant compound belonging to the stilbenes and is found in high concentrations in the skin of red grapes, berries, nuts and other plant products.
Resveratol according to the present invention contains natural resveratrol from the plant Polygonum cuspidatum which is known for its antioxidant activity.

There are several mechanisms that explain the pharmacological action of resveratrol. This substance acts against the enzyme cyclooxygenase COX-2 (IC50 = 32.2uM), cyclooxygenase COX-1 (IC50 = 15uM) and lipoxygenase (IC50 = 2.7 uM), inhibits the production of PGE2, NO and Caspase-3.

The composition of the present invention further comprises rosmarinic acid which is a phenolic plant component, ester of a caffeic acid. In addition to its proven antioxidant activity, rosmarinic acid has an action against inflammatory diseases as it has been shown by numerous in vitro and in vivo studies.

The composition of the present invention further comprises curcumin, a natural compound belonging to the class of curcuminoids and constituting the major substance of turmeric (Curcuma longa). Curcumin is one of the most effective anti-inflammatory plant ingredients through the IL-1β, TNF-α and MMPs pathways. Its effectiveness against the symptoms of osteoarthritis has been proven by clinical studies in patients. It helps in pain relieve, joint stiffness and muscle pain. However, it is known to have quite low bioavailability when taken per os (orally) as a dietary supplement. Therefore, in order to achieve the sufficient dose, it is used in the present invention in liposomal form, which has several times higher absorption from the gastrointestinal tract than the usual turmeric extracts.

Rosmarinic acid and curcumin have activity against MMPs metalloproteases with rosmarinic being a selective inhibitor of MMP13 (IC50 = 2.9uM), while curcumin has additional activity against TNF-α and IL-1β.

In one embodiment, the composition according to the present invention comprises, in addition to the active ingredients resveratrol, rosmarinic acid and curcumin, at least one further active ingredient of natural or synthetic origin with therapeutic action, or supplementary action, or otherwise useful for the proposed purposes of the present invention. Non-limiting examples of such active ingredients are vitamins and / or minerals, such as B complex vitamins; minerals, such as zinc and magnesium; antioxidants, such as ascorbic acid or vitamin C, and vitamin E. The additon of ascorbic acid in a content of more than 15% of the recommended daily allowance allows the use of the health claim with the verbal "Vitamin C contributes to the normal formation of collagen for the normal function of cartilage".

The composition of the present invention further may comprise vitamins and/or minerals, as vitamins and minerals can help reduce inflammation and rebuild joint cartilage. Examples of vitamins and minerals including, without limitation, ascorbic acid, pyridoxine chloride, gluthione, calcium citrate, magnesium citrate, magnesium oxide, calcium carbonate (e.g., lead-free calcium carbonate), zinc acetate, zinc gluconate and vitamin B complexes. Some antioxidants are water soluble, such as ascorbic acid or vitamin C, and some other antioxidants are fat-soluble, such as vitamin E.

In addition, the composition of the present invention may contain more than one vitamin. For example, the composition may contain two different vitamins. Likewise, the composition of the present invention may contain more than one mineral. For example, the composition of the present invention may contain two different minerals, such as zinc and magnesium.

In the composition according to the present invention, resveratrol is present in an amount by weight between 10% and 25% of the total weight of the composition, preferably between 12% and 18%, the rosmarinic acid is present in an amount by weight comprising between 10 % and 25% of the total weight of the composition, preferably between 12% and 20% and curcumin is present in an amount by weight ranging between 30% and 60%, preferably between 35% and 45% of the total weight of the composition. The composition of the present invention may contain any amount of vitamins and minerals according to the recommended daily allowance (RDA).
The pharmaceutical compositions of the present invention may also contain one or more additional formulation ingredients selected from a wide variety of excipients. According to the desired properties of the composition, any number of ingredients may be selected, alone or in combination, based on their known uses in the preparation of dietary supplement compositions.

Moreover, any excipient may optionally be added to the above composition, provided that they are compatible with the active ingredients of the composition, in order to overcome problems associated with unfavorable pharmacotechnical characteristics of these substances, and in order to increase the stability of the drug and shelf-life of the pharmaceutical product.
In some cases, the dietary supplement composition of the present invention may be substantially free of artificial colors, artificial flavors and chemical preservatives.
The pharmaceutical composition or dietary supplement composition or nutraceutical composition of the present invention will be prepared by conventional methods for obtaining immediate-release capsules or sachets that are well known to the skilled person in pharmaceutical technology. The capsules or sachets of the present invention may be prepared by mixing the active substances with one or more pharmaceutically acceptable excipients.

Another embodiment of the present invention is the use of a process for the preparation of a pharmaceutical composition or dietary supplement composition or nutraceutical for oral administration comprising a therapeutically effective amount of resveratrol or a pharmaceutically acceptable derivative thereof, in combination with a therapeutically effective amount of rosmarinic acid and at least one additional compound, such as curcumin, for the reduction of osteoarthritic pain and inflammation. The process steps may be as follows:
a) Weighing the individual active substances and their extracts, namely Polygonum Cuspidatum extract (resveratrol), rosemary extract (rosmarinic acid), turmeric (curcumin), ascorbic acid (vitamin C), and excipients, such as magnesium stearate, and microcrystalline cellulose;
b) Mixing all said active substances together with the excipients, such as magnesium stearate and microcrystalline cellulose for appropiate time so as to form a homogenous mixture.
c) Adding all said active substances in a blender and mixing for appropiate time until completely uniform.
d) Filling hard capsules or sachets
e) Packaging and quality control.

All percentages stated herein are weight percentages based on total composition weight, unless otherwise stated.

The following examples illustrate preferred embodiments in accordance with the present invention without limiting the scope or spirit of the invention:

### EXAMPLES

### Example 1: Dietary Supplement Composition 1 of the present invention

Preferred pharmaceutical or dietary supplement composition 1 according to the present invention is illustrated in Table 1.

**TABLE 1:**

| | **Ingredients** | **Weight (mg)** | **% of total weight** |
|---|---|---|---|
| 1 | Resveratrol | 51.9 | 13.33 |
| 2 | Rosmarinic acid | 51.9 | 13.33 |
| 3 | Curcumin | 148.4 | 38.11 |
| 4 | Vitamin C | 29.7 | 7.63 |
| 5 | Microcrystalline Cellulose | 100.5 | 25.81 |
| 6 | Magnesium stearate | 7.0 | 1.80 |
| | **Total** | 389.4 | 100.00 |

The capsules of the present invention are from plant origin, size 0 (the capsule weight is about 109.0 ± 6 mg) with pharmaceutically acceptable ingredients. They have a decomposition time of less than 15 minutes and moisture percentages between 4 and 8%.

The pharmaceutical composition or dietary supplement composition or nutraceutical composition 1 of the present invention was prepared according to the following preparation process:
All ingredients were weighted and the individual active substances and extracts were placed in a blender together with the excipients such as binder, filler, etc. until complete homogenization. The mixture was used to fill hard capsules or sachets. The capsules or sachets were then packaged while a quality control was performed.
Curcumin used in composition 1 of the present invention was in phospholipid form. The ratio of resveratrol to rosmarinic acid used in the composition 1 of the present invention is from 1: 0.5 to 1: 1.5, preferably from 1: 1 to 1: 3 and more preferably 1: 1.

All tests were performed in accordance with European Pharmacopoeia and were within the specifications.
Another object of the present invention was to prepare a pharmaceutical composition which is stable for a long period of storage.

The pharmaceutical composition or dietary supplement composition or nuttaceutical of the present invention is specifically designed to support patients with osteoarthritis who are in the early or advanced stages, in combination or not with other pharmaceutical treatment.

The advantage of the present invention lies in the synergistic - complementary action of natural ingredients as inhibitors of the main inflammatory mechanisms in osteoarthritis.
The product of the present invention, with said combination of ingredients, has no direct competition in the market. Most osteoarthritic supplements are based on the ingredients glucosamine, chondroitin sulfate, and combination thereof.

While the present invention has been described with respect to the particular embodiments, it will be apparent to those skilled in the art that various changes and modifications may be made in the invention without departing from the spirit and scope thereof, as defined in the appended claims.

## Claims

1. A pharmaceutical composition or dietary supplement composition or nutraceutical composition comprising a therapeutically effective amount of resveratrol or a pharmaceutically acceptable derivative thereof, and a therapeutically effective amount of rosmarinic acid and at least one additional compound, such as curcumin, to be used for the reduction of osteoarthritic pain and inflammation.

2. The pharmaceutical composition or dietary supplement composition or nutraceutical composition according to claim 1, wherein the ratio of resveratrol to rosmarinic acid is from 1: 0.5 to 1:1.5, preferably from 1:1 to 1:3 and most preferably 1:1.

3. The pharmaceutical composition or dietary supplement composition or nutraceutical composition according to claim 1 or 2, wherein said at least one additional compound is curcumin in a phospholipid or another liposomal form.

4. The pharmaceutical composition or dietary supplement composition or nutraceutical composition according to claim 1 or 2 or 3, wherein further comprises vitamins such as ascorbic acid or vitamin C.

5. The pharmaceutical composition or dietary supplement composition or nutraceutical composition according to any preceding claim, wherein the amount of resveratrol in said composition is in the range of 10 - 25% by weight percentage % of the total weight of the composition, and the amount of rosmarinic acid in said composition is in the range of 10 - 25% by weight in percentage % of the total weight of the composition and the amount of curcumin in said composition is in the range of 30 ― 60% by weight percentage % of the total weight of the composition.

6. The pharmaceutical composition or dietary supplement composition or nutraceutical composition according to claim 4, wherein it further comprises vitamins of B complex and vitamin E, and minerals, such as magnesium and zinc.

7. The pharmaceutical composition or dietary supplement composition or nutraceutical composition according to any preceding claim, wherein it further comprises pharmaceutically acceptable excipients selected from a group comprising of absorbents, glidants, diluents, binders, chelating agents, coating agents, pigments, complexing agents, solubilizers, emollients, emulsifiers, fillers, gelling agents, lubricants, moisturizers, retarding agents, stabilizers, suspending agents, sweeteners, thickening agents, surfactants, opacifiers, coloring agents, antigellants, rheology control agents, and their combinations thereof.

8. A process for the preparation of a pharmaceutical composition or dietary supplement composition or nutraceutical comprising a therapeutically effective amount of resveratrol or a pharmaceutically acceptable derivative thereof, in combination with a therapeutically effective amount of rosmarinic acid and at least one additional compound, such as curcumin, for the reduction of osteoarthritic pain and inflammation, wherein said process comprises following steps:
a) Weighing the individual active substances and their extracts, namely Polygonum Cuspidatum extract, rosemary extract, turmeric, ascorbic acid, and excipients, such as magnesium stearate and microcrystalline cellulose.
b) Mixing all said active substances together with the excipients, such as magnesium stearate and microcrystalline cellulose for appropiate time so as to form a homogenous mixture.
c) Adding all said active substances in a blender and mixing for appropiate time until completely uniform.
d) Filling hard capsules or sachets
e) Packaging and quality control.

9. The process for the preparation of a pharmaceutical composition or dietary supplement composition or nutraceutical composition according to claim 8, wherein said curcumin is in a phospholipid or another liposomal form.
